# EUROPÄISCHE PATENTANMELDUNG

(11) **EP 0 549 879 A2**
(43) Veröffentlichungstag der Anmeldung: **07.07.1993**
(21) Anmeldenummer: 92119778.6
(22) Anmeldetag: 20.11.1992
(51) Int. Cl.: C07D 417/12, A61K 31/47

(54) **Thiazolyl-substituierte Chinolylmethoxyphenylessigsäurederivate**

(30) Priorität: 03.12.1991 DE 4139751
(71) Anmelder: BAYER AG, 51368 Leverkusen (DE)
(72) Erfinder: Raddatz, Siegfried, Dr., W-5000 Köln 80 (DE); Mohrs, Klaus-Helmut, Dr., W-5600 Wuppertal 1 (DE); Matzke, Michael, Dr., W-5600 Wuppertal 1 (DE); Fruchtmann, Romanis, Dr., W-5000 Köln 60 (DE); Hatzelmann, Armin, Dr., W-7750 Konstanz (DE); Müller-Peddinghaus, Reiner, Prof. Dr., W-5060 Bergisch Gladbach 2 (DE)

(57) **Zusammenfassung**

Thiazolyl-substituierte Chinolylmethoxyphenylessigsäurederivate der allgemeinen Formel 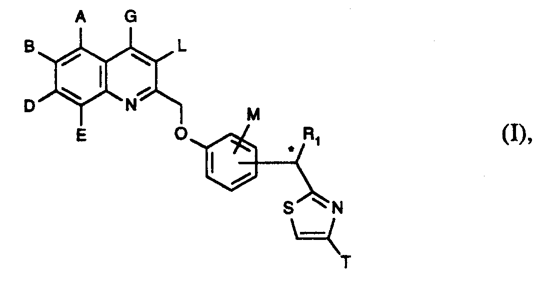
in welcher
- A,B,D,E,G,L und M: gleich oder verschieden sind und für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist,
- R¹: für geradkettiges oder verzweigtes Alkyl mit bis zu 8 Kohlenstoffatomen steht, das gegebenenfalls durch Phenyl oder Cycloalkyl mit 3 bis 12 Kohlenstoffatomen stubstituiert ist, oder für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
- T: für geradkettiges oder verzweigtes Alkyl mit bis zu 6 Kohlenstoffatomen oder für die Gruppe der Formel 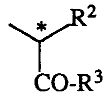 steht, worin
- R²: Wasserstoff oder geradkettiges oder verzweigtes Alkyl mit bis zu 8 Kohlenstoffatomen bedeutet, oder Benzyl oder Cycloalkyl mit 3 bis 12 Kohlenstoffatomen bedeutet.
- R³: einen Rest der Formel -OR⁴ oder -NR⁵-SO₂-R⁶ bedeutet,
worin
R⁴ Wasserstoff, geradkettiges oder verzweigtes Alkyl mit bis zu 8 Kohlenstoffatomen oder Phenyl bedeutet,
R⁵ Wasserstoff oder geradkettiges oder verzweigtes Alkyl mit bis zu 6 Kohlenstoffatomen oder Benzyl bedeutet,
R⁶ Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert ist,
   oder
   geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,
gegebenenfalls in einer isomeren Form, und deren Salze.

Die Stoffe können als Hemmer von enzymatischen Reaktionen im Rahmen des Arachidonsäuremetabolismus in Arzneimitteln eingesetzt werden.

## Beschreibung

Die Erfindung betrifft Thiazolyl-substituierte Chinolylmethoxyphenylessigsäurederivate, Verfahren zu ihrer Herstellung und ihre Verwendung in Arzneimitteln.

In der EP 200 101 A2 sind Aryl- und Heteroarylether mit einer Lipoxygenase inhibierenden, antiinflammatorischen und anti-allergischen Wirkung beschrieben.

Außerdem sind substituierte 4-(Chinolin-2-yl-methoxy)phenylessigsäurederivate und α-substituierte 4-(Chinolin-2-yl-methoxy)phenylessigsäurederivate aus EP 344 519 (US 4 970 215) und EP 339 416 bekannt.

Ferner wird in der EP 351 194 A2 ein Verfahren zur Herstellung von lipoxygenaseinhibierenden Aryl-substituierten Thiazolen beschrieben.

Die vorliegende Erfindung betrifft jetzt Thiazolyl-substituierte Chinolylmethoxyphenylessigsäurederivate der allgemeinen Formel (I) 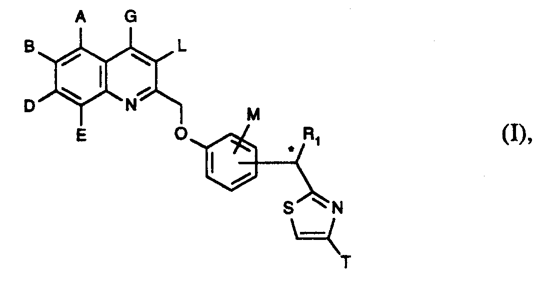
in welcher
- A, B, D, E, G, L und M: gleich oder verschieden sind und
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
- R¹: für geradkettiges oder verzweigtes Alkyl mit bis zu 8 Kohlenstoffatomen steht, das gegebenenfalls durch Phenyl oder Cycloalkyl mit 3 bis 12 Kohlenstoffatomen substituiert ist, oder
für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
- T: für geradkettiges oder verzweigtes Alkyl mit bis zu 6 Kohlenstoffatomen oder für die Gruppe der Formel 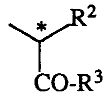 steht,
worin
- R²: Wasserstoff oder geradkettiges oder verzweigtes Alkyl mit bis zu 8 Kohlenstoffatomen bedeutet oder
Benzyl oder Cycloalkyl mit 3 bis 12 Kohlenstoffatomen bedeutet
und
- R³: einen Rest der Formel -OR⁴ oder -NR⁵-SO₂-R⁶ bedeutet,
worin
- R⁴: Wasserstoff, geradkettiges oder verzweigtes Alkyl mit bis zu 8 Kohlenstoffatomen oder Phenyl bedeutet,
- R⁵: Wasserstoff oder geradkettiges oder verzweigtes Alkyl mit bis zu 6 Kohlenstoffatomen oder Benzyl bedeutet,
- R⁶: Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert ist, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,
gegebenenfalls in einer isomeren Form,
und deren Salze.

Im Rahmen der vorliegenden Erfindung werden physiologisch unbedenkliche Salze bevorzugt. Physiologisch unbedenkliche Salze der Thiazolyl-substituierten Chinolylmethoxyphenylessigsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen existieren in stereoisomeren Formen (*), die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B, D, E, G, L und M: gleich oder verschieden sind und
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,
- R¹: für geradkettiges oder verzweigtes Alkyl mit bis zu 6 Kohlenstoffatomen steht, das gegebenenfalls durch Phenyl, Cyclopropyl, Cyclopentyl, Cyclohexyl oder Cycloheptyl substituiert ist, oder
für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 6 Kohlenstoffatomen substituiert sind,
- T: für geradkettiges oder verzweigtes Alkyl mit bis zu 4 Kohlenstoffatomen oder für die Gruppe der Formel 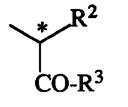 steht,
worin
- R²: Wasserstoff oder geradkettiges oder verzweigtes Alkyl mit bis zu 6 Kohlenstoffatomen bedeutet, oder
Benzyl, Cyclopropyl, Cyclobutyl, Cyclopentyl, Cyclohexyl oder Cycloheptyl bedeutet
und
- R³: einen Rest der Formel -OR⁴ oder -NR⁵-SO₂-R⁶ bedeutet,
worin
- R⁴: Wasserstoff oder geradkettiges oder verzweigtes Alkyl mit bis zu 6 Kohlenstoffatomen bedeutet,
- R⁵: Wasserstoff oder geradkettiges oder verzweigtes Alkyl mit bis zu 4 Kohlenstoffatomen bedeutet,
- R⁶: Phenyl bedeutet, das gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder
geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann,
gegebenenfalls in einer isomeren Form,
und deren Salze.

Besonders bevorzugt sind Verbindungen der allgemeinen Formel (I),
in welcher
- A, B, D, E, G, L und M: gleich oder verschieden sind und
für Wasserstoff, Hydroxy, Fluor, Chlor, Brom oder geradkettiges oder verzweigtes Alkyl mit bis zu 4 Kohlenstoffatomen stehen,
- R¹: für geradkettiges oder verzweigtes Alkyl mit bis zu 6 Kohlenstoffatomen setht, das gegebenenfalls durch Phenyl, Cyclopropyl, Cyclopentyl oder Cyclohexyl substituiert ist oder
für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 4 Kohlenstoffatomen substituiert sind, T für Methyl oder für die Gruppe der Formel 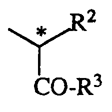 steht,
worin
- R²: Wasserstoff oder geradkettiges oder verzweigtes Alkyl mit bis zu 4 Kohlenstoffatomen bedeutet,
und
- R³: einen Rest der Formel -OR⁴ oder -NR⁵-SO₂-R⁶ bedeutet, worin
- R⁴: Wasserstoff oder geradkettiges oder verzweigtes Alkyl mit bis zu 4 Kohlenstoffatomen bedeutet,
- R⁵: Wasserstoff, Methyl oder Ethyl bedeutet,
- R⁶: Phenyl bedeutet, das gegebenenfalls durch Fluor, Chlor, Brom, Jod, Methoxy oder Trifluormethyl substituiert ist, oder geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann,
gegebenenfalls in einer isomeren Form,
und deren Salze.

Ganz besonders bevorzugt sind Verbindungen der allgemeinen Formel (I),
in welcher
- A, B, D, E, G, L und M: für Wasserstoff stehen.

Ebenso sind solche Verbindungen ganz besonders bevorzugt, in denen der Rest 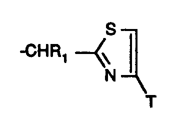
in 4-Stellung zum Chinolylmethoxyrest steht.

Außerdem wurde ein Verfahren zur Herstellung der erfindungsgemäßen Verbindungen der allgemeinen Formel (I) gefunden, dadurch gekennzeichnet, daß man
Thioamide der allgemeinen Formel (II) 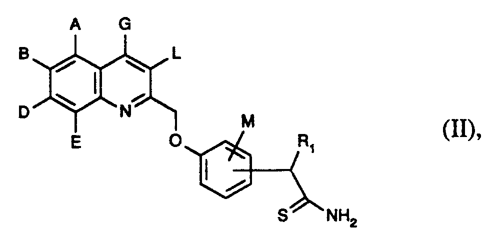
in welcher
- A, B, D, E, G, L, M: und R¹ die oben angegebene Bedeutung haben,
mit Halogenacetessigesterderivaten oder Halogenketonen der allgemeinen Formel (III)

Cl-CH₂-CO-CH₂-W (III),

in welcher
- W: für Wasserstoff, C₁-C₆-Alkyl oder für die Gruppe -CO-R⁷ steht,
worin

R⁷ C₁-C₄-Alkoxy bedeutet

in organischen Lösemitteln, gegebenenfalls in Anwesenheit von Wasser, umsetzt,
und im Fall der Säuren (R³ = OH) die Ester nach üblicher Methode verseift,
und im Fall, daß R² nicht für Wasserstoff steht, eine Alkylierung anschließt,
und im Fall der Sulfonamide (R³ = -NH-SO₂R⁶) eine Amidierung in inerten Lösemitteln, gegebenenfalls in Anwesenheit einer Base und/oder eines Hilfsstoffes, durchführt, wobei die Substituenten A, B, D, E, G, L und M auf jeder der oben aufgeführten Stufen nach literaturbekannten Methoden variiert werden können.

Das erfindungsgemäße Verfahren kann durch folgendes Formelschema beispielhaft erläutert werden: 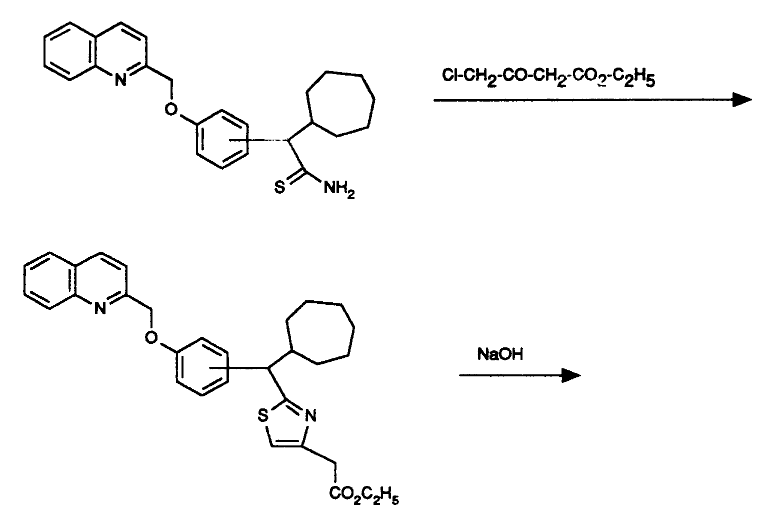 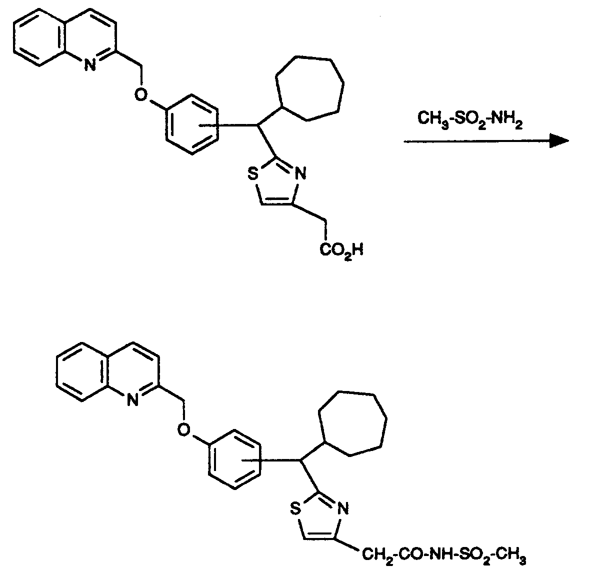
Als Lösemittel für die einzelnen Reaktionsschritte eignen sich im allgemeinen inerte organische Lösemittel, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen. Für die Umsetzung mit den Verbindungen der allgemeinen Formel (II) ist Toluol bevorzugt.

Das Verfahren wird im allgemeinen in einem Temperaturbereich von 0°C bis 150°C, bevorzugt bei 25°C bis 40°C, durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0°C bis +150°C, vorzugsweise bei Raumtemperatur bis +100°C, bei Normaldruck durchgeführt.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, Trichlorethylen oder Kohlenwassserstoffe wie Benzol, Xylol, Toluol, Hexan, Cyclohexan oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt Es ist aber auch möglich, das Verfahren bei Unterdruck oder bei Überdruck durchzuführen (z.B. in einem Bereich von 0,5 bis 5 bar).

Bei der Durchführung der Amidierung wird die Base im allgemeinen in einer Menge von 1 bis 3 Mol, bevorzugt von 1 bis 1,5 Mol, bezogen auf 1 Mol der Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochiorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int Pept. Prot. Res. 13, 403 (1979), 17, 187 (1981)].

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters, eingesetzt. Besonders bevorzugt verwendet man molare Mengen der Reaktanden.

Die Verbindungen der allgemeinen Formel (II) sind größtenteils neu und können hergestellt werden, indem man
Amide der allgemeinen Formel 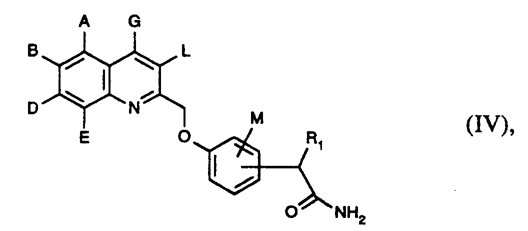
in welcher
- A, B, D, E, G, L, M: und R¹ die angegebene Bedeutung haben,
mit Lawesson's Reagens der Formel (V) 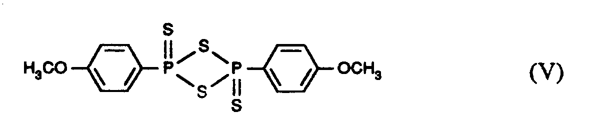
in inerten Losemitteln, gegebenenfalls unter Schutzgasatmosphäre,
umsetzt.

Die Verbindungen der allgemeinen Formel (IV) werden partiell als Zwischenprodukte vom Bedeutungsumfang der EP 399 291 erfaßt, als konkrete Stoffvertreter aber neu und können beispielsweise hergestellt werden, indem man
Verbindungen der allgemeinen Formel (VI) 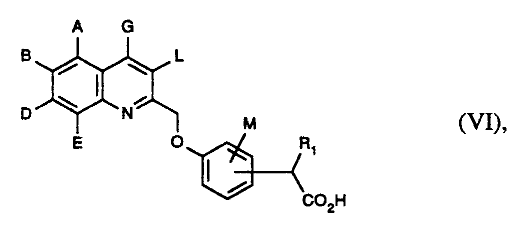
in welcher
- A, B, D, E, G, L, M: und R¹ die oben angegebene Bedeutung haben,
mit Carbonyldiimidazol in inerten Lösemitteln, gegebenenfalls in Anwesenheit einer der oben aufgeführten Basen, zu den Verbindungen der allgemeinen Formel (VII) 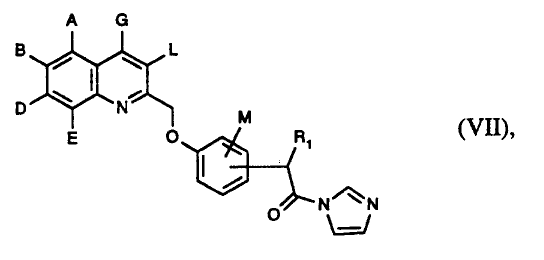
in welcher
- A, B, D, E, G, L, M: und R¹ die oben angegebene Bedeutung haben,
umsetzt und anschließend in inerten Lösemitteln, in Anwesenheit von Ammoniumchlorid und Ammoniak die Amide herstellt.

Als Lösemittel eignen sich die oben aufgeführten Lösemittel. Bevorzugt ist für beide Verfahrensschritte Tetrahydrofuran.

Im allgemeinen wird das Verfahren bei Normaldruck durchgeführt. Es ist aber auch möglich, bei Unterdruck oder bei Überdruck zu arbeiten (z.B. von 0,5 bis 5 bar).

Das Verfahren wird im allgemeinen in einem Temperaturbereich von 0°C bis +100°C, bevorzugt von +15°C bis +70°C, durchgeführt.

Die Verbindungen der allgemeinen Formel (VI) sind bekannt [vgl. US 4 970 715].

Die Verbindungen der allgemeinen Formel (VII) sind neu und können nach den oben aufgeführten Verfahren hergestellt werden.

Die Verbindung der allgemeinen Formel (V) ist bekannt (vgl. MSD 2,2069 B].

Die Verbindungen der allgemeinen Formel (II) sind teilweise bekannt oder neu und können in diesem Fall nach der oben aufgeführten Methode hergestellt werden.

Chloracetessigsäurederivate der allgemeinen Formel (III) sind bekannt [vgl. z.B. Beilstein 3,663, Teil 1,653].

Die reinen Enantiomeren der erfindungsgemäßen Verbindungen der allgemeinen Formel (I) können beispielsweise hergestellt werden, indem man die entsprechenden enantiomerenreinen Säuren nach üblicher Methode trennt und anschließend wie oben aufgeführt weiter umsetzt.

Die erfindungsgemäßen Verbindungen können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Beispielsweise zeigen die Verbindungen der allgemeinen Formel (I) überraschenderweise eine hohe in vitro Aktivität als Leukotriensynthesehemmer und eine starke in vivo Wirkung nach oraler Applikation.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündunge/Rheuma und Ödemen, Thrombosen und Thromboembolien, Ischämien (periphere, cardiale, cerebrale Durchblutungsstörungen), Herz-und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen Verbindungen können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die 5-Lipoxygenase-Hemmung in vitro wurde die Freisetzung von Leukotrien B₄ (LTB₄) an polymorphkernigen Humanleukozyten (PMN) nach Zugabe von Substanzen und Ca-Ionophor mittels reverse phase HPLC nach Borgeat, P. et al., Proc. Nat. Acad. Sci. 76,2148-2152(1979), bestimmt.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

### 4-(2-Chinolinylmethoxy)phenyl-cycloheptylessigsäure-imidazolid

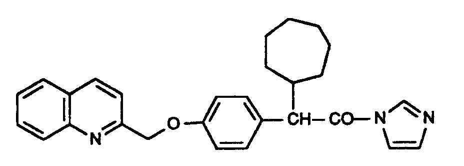

409 g (2,1 mol) Säure und 186,5 g (2,3 mol) Carbonyldiimidazol wurden bei Raumtemperatur in 5 l Tetrahdyrofuran gerührt. Nach Abklingen der ersten CO₂-Entwicklung wurde bis zur vollständigen Umsetzung (HPLC-Kontrolle / DC) bei 40°C gerührt. Danach wurde das Lösemittel abdestilliert Der Rückstand wurde in 2 l Isopropanol heiß gelöst und mit 2 l Wasser versetzt Der Niederschlag wurde abgesaugt, mit 1/2 l Isopropanol/H₂O (1:1) gewaschen und anschließend nochmal mit 1 l Wasser gewaschen. Es wurde im Umlufttrockenschrank bei 50°C getrocknet
Ausbeute: 417 g (90% der Theorie)
F°C = 146-148°C

### Beispiel II

### 4-(2-Chinolinylmethoxy)phenyl-cycloheptyl-acetamid

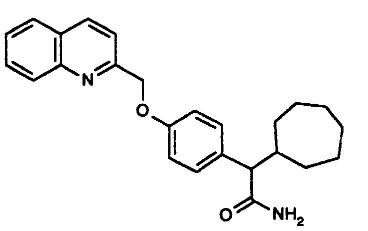

5 g (0,011 mol) der Verbindung aus Beispiel I und eine Spatelspitze NH₄Cl wurden in 100 ml THF gelöst und auf ca. 55°C erwärmt. Bei dieser Temperatur leitete man 4 h einen Ammoniakstrom durch die Lösung. Anschließend ließ man erkalten, engt auf ein kleines Volumen ein und verrührte den Rückstand mit Dichlormethan.
Ausbeute: 4,1 g (92,8% der Theorie) farblose Kristalle
Fp.: 173°C

### Beispiel III

### 4-(2-Chinolinylmethoxy)phenyl-cycloheptyl-thioacetamid

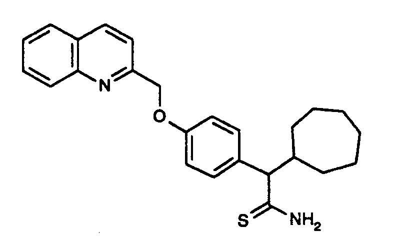

2,0 g (0,0052 mol) der Verbindung aus Beispiel II und 1,3 g (0,0031 mol) Lawesson's Reagens wurden mit 15 ml Toluol unter Argonatmosphäre 3 h zum Sieden erhitzt. Anschließend erfolgte eine säulenchromatographische Trennung (Kieselgel 60, Toluol/THF 100:5). Man erhielt (R_{f} = 0,45) ein leicht gelbliches Produkt, das sich aus Diisopropylether umkristallisieren ließ.
Ausbeute: 0,9 g (43,2% der Theorie)
Fp.: 137°C

### Herstellungsbeispiele

### Beispiel 1

### 2-[4-(2-Chinolinylmethoxy)phenylcycloheptylmethyl]-4-methyl-thiazol

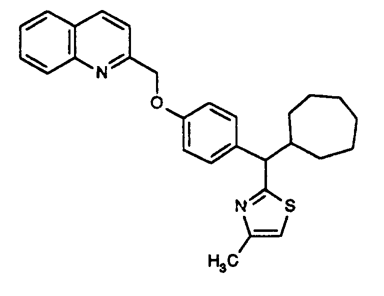

1,5 g (0,0037 mol) der Verbindung aus Beispiel III und 5 ml Chloraceton werden in einem Ölbad mit 10 ml Wasser 30 Minuten auf 100°C erhitzt. Anschließend neutralisiert man mit Natriumhydrogencarbonatlösung und extrahiert mit Dichlormethan. Nach Trocknen mit Natriumsulfat engt man i.V. zur Trockne ein und rekristalliert den Rückstand am Diisopropylether.
Ausbeute: 1,3g (79% d. Th.)
farblose Kristalle, Fp.: 119°C

### Beispiel 2

### 2-[4-(2-Chinolinylmethoxy)phenylcycloheptylmethyl]-thiazol-4-essigsäureethylester

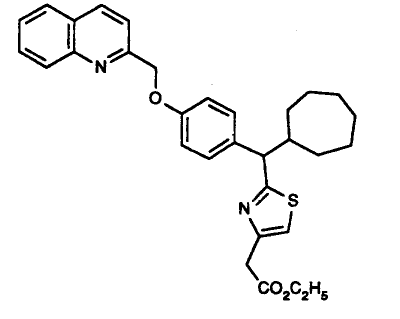

1,5 g (0,0037 mol) der Verbindung aus Beispiel III und 1,5 ml = 1,83 g (0,011 mol) Chloracetessigsäureethylester wurden 1 h zusammen mit 30 ml Toluol und 10 ml Wasser auf 100°C erhitzt. Man neutralisierte mit 10%iger Natriumhydrogencarbonat-Lösung, trennte die organische Phase ab, trocknete mit Natriumsulfat, engte i.V. auf ein Kleines Volumen ein und trennte säulenchromatographisch. Man erhielt ein gelbes Öl.
Ausbeute: 1,5 g (78,6% der Theorie)

### Beispiel 3

### 2-[4-(2-Chinolinylmethoxy)-phenylcycloheptylmethyl]-thiazol-4-essigsäure

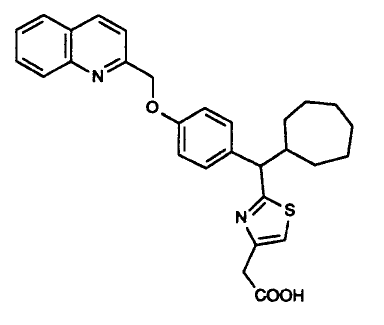

1,4 g (0,00272 mol) der Verbindung aus Beispiel 2 wurden in 20 ml Ethanol gelöst, mit 5 ml 1 n Natronlauge versetzt und 2 h zum Sieden erhitzt. Nach dem Erkalten versetzte man mit 5 ml 1 N Salzsäure. Den angefallenen, farblosen Niederschlag filtrierte man ab und trocknete ihn. Das Produkt ist amorph.
Ausbeute: 1,3 g (98,2% der Theorie)

### Beispiel 4

### N-Methylsulfonyl-2-[4-(2-chinolinylmethoxy)phenylcycloheptylmethyl]thiazol-4-acetamid

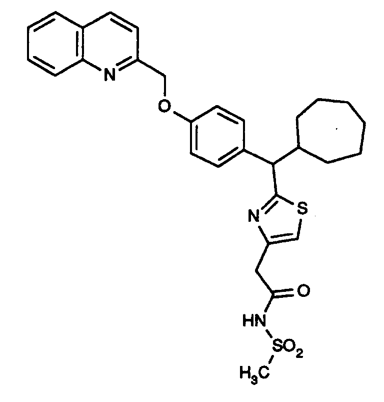

1,5 g (0,0031 mol) der Verbindung aus Beispiel 3, 0,38 g (0,004 mol) Methansulfonamid, 0,75 g (0,004 mol) N-Dimethylaminopropyl-N-ethyl-carbodiimidhydrochlorid und 0,75 g (0,0061 mol) Dimethylaminopyridin (DMAP) wurden in 30 ml trockenem Dichlormethan gelöst und über Nacht bei Raumtemperatur gerührt. Anschließend extrahierte man 2 mal mit 30 ml Wasser, trocknete die organische Phase mit Natriumsulfat, engte im Vakuum auf ein kleines Volumen ein und trennte den Rest säulenchromatographisch.
R_{f} = 0,8 (nach Abdampfen des Lösemittels: farbloser Schaum)
Ausbeute: 57,6% der Theorie

## Patentansprüche

1. Thiazolyl-substituierte Chinolylmethoxyphenylessigsäurederivate der allgemeinen Formel 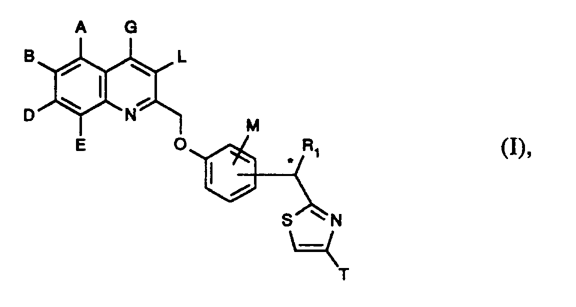 in welcher
A, B, D, E, G, L und M gleich oder verschieden sind und
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
R¹ für geradkettiges oder verzweigtes Alkyl mit bis zu 8 Kohlenstoffatomen steht, das gegebenenfalls durch Phenyl oder Cycloalkyl mit 3 bis 12 Kohlenstoffatomen substituiert ist, oder
für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
T für geradkettiges oder verzweigtes Alkyl mit bis zu 6 Kohlenstoffatomen oder für die Gruppe der Formel 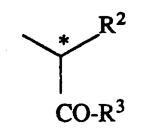 steht,
worin
R² Wasserstoff oder geradkettiges oder verzweigtes Alkyl mit bis zu 8 Kohlenstoffatomen bedeutet, oder
Benzyl oder Cycloalkyl mit 3 bis 12 Kohlenstoffatomen bedeutet.
R³ einen Rest der Formel -OR⁴ oder -NR⁵-SO₂-R⁶ bedeutet,
worin
R⁴ Wasserstoff, geradkettiges oder verzweigtes Alkyl mit bis zu 8 Kohlenstoffatomen oder Phenyl bedeutet,
R⁵ Wasserstoff oder geradkettiges oder verzweigtes Alkyl mit bis zu 6 Kohlenstoffatomen oder Benzyl bedeutet,
R⁶ Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert ist, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,
gegebenenfalls in einer isomeren Form,
und deren Salze.

2. Thiazolyl-substituierte Chinolylmethoxyphenylessigsäurederivate nach Anspruch 1, wobei
A, B, D, E, G, L und M gleich oder verschieden sind und
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,
R¹ für geradkettiges oder verzweigtes Alkyl mit bis zu 6 Kohlenstoffatomen steht, das gegebenenfalls durch Phenyl, Cyclopropyl, Cyclopentyl, Cyclohexyl oder Cycloheptyl substituiert ist, oder
für Cyclopropyl, Cyclobutyl, Cyclopentyl, Cyclohexyl, Cycloheptyl oder Cyclooctyl steht, die gegebenenfalls durch geradkettiges oder verzweigtes Akyl mit bis zu 6 Kohlenstoffatomen substituiert sind,
T für geradkettiges oder verzweigtes Alkyl mit bis zu 4 Kohlenstoffatomen oder für die Gruppe der Formel 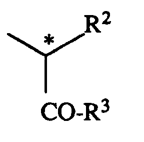 steht,
worin
R² Wasserstoff oder geradkettiges oder verzweigtes Alkyl mit bis zu 6 Kohlenstoffatomen bedeutet, oder
Benzyl, Cyclopropyl, Cyclobutyl, Cyclopentyl, Cyclohexyl oder Cycloheptyl bedeutet,
R³ einen Rest der Formel -OR⁴ oder -NR⁵-SO₂-R⁶ bedeutet,
worin
R⁴ Wasserstoff oder geradkettiges oder verzweigtes Alkyl mit bis zu 6 Kohlenstoffatomen bedeutet,
R⁵ Wasserstoff oder geradkettiges oder verzweigtes Alkyl mit bis zu 4 Kohlenstoffatomen bedeutet,
R⁶ Phenyl bedeutet, das gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann,
gegebenenfalls in einer isomeren Form,
und deren Salze.

3. Thiazolyl-substituierte Chinolylmethoxyphenylessigsäurederivate nach Anspruch 1, wobei
A, B, D, E, G, L und M gleich oder verschieden sind und
für Wasserstoff, Hydroxy, Fluor, Chlor, Brom oder geradkettiges oder verzweigtes Alkyl mit bis zu 4 Kohlenstoffatomen stehen,
R¹ für geradkettiges oder verzweigtes Alkyl mit bis zu 6 Kohlenstoffatomen steht, das gegebenenfalls durch Phenyl, Cyclopropyl, Cyclopentyl oder Cyclohexyl substituiert ist oder
für Cyclopropyl, Cyclobutyl, Cyclopentyl, Cyclohexyl, Cycloheptyl oder Cyclooctyl steht, die gegebenenfalls durch geradkettiges oder verzweigtes Alkyl mit bis zu 4 Kohlenstoffatomen substituiert sind,
T für Methyl oder für die Gruppe der Formel 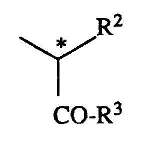 steht,
worin
R² Wasserstoff oder geradkettiges oder verzweigtes Alkyl mit bis zu 4 Kohlenstoffatomen bedeutet,
R³ einen Rest der Formel -OR⁴ oder -NR⁵-SO₂-R⁶ bedeutet,
worin
R⁴ Wasserstoff oder geradkettiges oder verzweigtes Alkyl mit bis zu 4 Kohlenstoffatomen bedeutet,
R⁵ Wasserstoff, Methyl oder Ethyl bedeutet,
R⁶ Phenyl bedeutet, das gegebenenfalls durch Fluor, Chlor, Brom, Jod, Methoxy oder Trifluormethyl substituiert ist, oder
geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann,
gegebenenfalls in einer isomeren Form,
und deren Salze.

4. Thiazolyl-substituierte Chinolylmethoxyphenylessigsäurederivate nach Anspruch 1, wobei
A, B, D, E, G, L und M für Wasserstoff stehen.

5. Thiazolyl-substituierte Chinolyhmethoxyphenylessigsäurederivate nach Anspruch 1, wobei der Rest der Formel 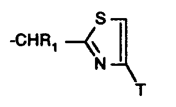 in 4-Stellung zum Chinolylmethoxyrest steht.

6. Thiazolyl-substituierte Chinolylmethoxyphenylessigsäurederivate nach Anspruch 1 zur Behandlung von Krankheiten.

7. Verfahren zur Herstellung von Thiazolyl-substituierten Chinolylmethoxyphenylessigsäurederivaten der allgemeinen Formel 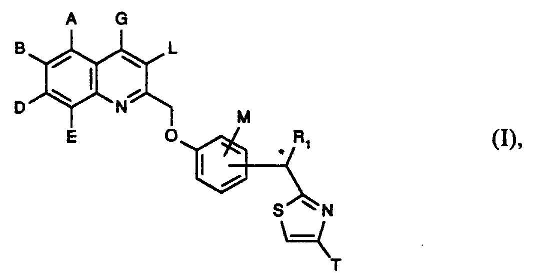 in welcher
A, B, D, E, G, L und M gleich oder verschieden sind und
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
R¹ für geradkettiges oder verzweigtes Alkyl mit bis zu 8 Kohlenstoffatomen steht, das gegebenenfalls durch Phenyl oder Cycloalkyl mit 3 bis 12 Kohlenstoffatomen substituiert ist, oder für Cycloalkyl mit 3 bis 12 Kohlenstoffatomen steht, das gegebenenfalls durch geradkettiges oder verzweigtes Alkyl mit bis zu 8 Kohlenstoffatomen substituiert ist,
T für geradkettiges oder verzweigtes Alkyl mit bis zu 6 Kohlenstoffatomen oder für die Gruppe der Formel 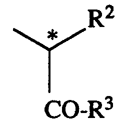 steht,
worin
R² Wasserstoff oder geradkettiges oder verzweigtes Alkyl mit bis zu 8 Kohlenstoffatomen bedeutet, oder
Benzyl oder Cycloalkyl mit 3 bis 12 Kohlenstoffatomen bedeutet
und
R³ einen Rest der Formel -OR⁴ oder -NR⁵-SO₂-R⁶ bedeutet,
worin
R⁴ Wasserstoff, geradkettiges oder verzweigtes Alkyl mit bis zu 8 Kohlenstoffatomen oder Phenyl bedeutet,
R⁵ Wasserstoff oder geradkettiges oder verzweigtes Alkyl mit bis zu 6 Kohlenstoffatomen oder Benzyl bedeutet,
R⁶ Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert ist, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,
gegebenenfalls in einer isomeren Form,
und deren Salze,
dadurch gekennzeichnet, daß man
Thioamide der allgemeinen Formel (II) 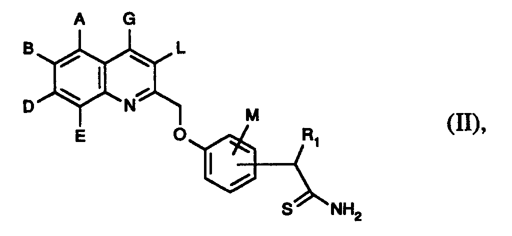 in welcher
A, B, D, E, G, L, M und R¹ die oben angegebene Bedeutung haben,
mit Halogenacetessigesterderivaten oder Halogenketonen der allgemeinen Formel (III)
Cl-CH₂-CO-CH₂-W (III),
in welcher
W für Wasserstoff, C₁-C₆-Alkyl oder für die Gruppe der Formel - CO-R⁷ steht,
worin
R⁷ C₁-C₄-Alkoxy bedeutet,
in organischen Lösemitteln, gegebenenfalls in Anwesenheit von Wasser, umsetzt,
und im Fall der Säuren (R³ = OH) die Ester nach üblicher Methode verseift,
und im Fall, daß R² nicht für Wasserstoff steht, eine Alkylierung anschließt,
und im Fall der Sulfonamide (R³ = -NH-SO₂R⁶) eine Amidierung in inerten Lösemitteln, gegebenenfalls in Anwesenheit einer Base und/oder eines Hilfsstoffes, durchführt, wobei die Substituenten A, B, D, E, G, L und M auf jeder der oben aufgeführten Stufen nach literaturbekannten Methoden variiert werden können.

8. Arzneimittel enthaltend mindestens ein Thiazolyl-substituiertes Chinolylmethoxyphenylessigsäurederivat nach Anspruch 1.

9. Arzneimittel nach Anspruch 8 zur Behandlung und Verhütung von Erkrankungen der Atemwege, wie Allergien/Asthma, Bronchitis, Emphysema, Schocklunge, pulmonaler Hypertonie, Entzündunge/Rheuma und Ödemen, Thrombosen und Thromboembolien, Ischämien, Herz- und Hirninfarkten, Herzrhythmusstörungen, Angina pectoris, Arteriosklerose, Dermatosen, Infektionen der Haut durch Bakterien, Metastasen und zur Cytoprotection im Gastrointestinaltrakt.

10. Verwendung von Thiazolyl-substituierten Chinolylmethoxyphenylessigsäurederivaten nach Anspruch 1 zur Herstellung von Arzneimitteln.
